# EUROPEAN PATENT APPLICATION

(11) **EP 4 413 981 A1**
(43) Date of publication of application: **14.08.2024**
(21) Application number: 22878942.6
(22) Date of filing: 07.10.2022
(51) Int. Cl.: A61K 31/343, A61P 43/00, A61P 1/16, A61P 11/00, A61P 35/00, C07D 307/80

(54) **NOVEL BENZOFURANYL HYDROXYPHENYL METHANONE DERIVATIVE COMPOUND OR PHARMACEUTICALLY ACCEPTABLE SALT THEREOF**

(30) Priority: 07.10.2021 KR 20210132806
(71) Applicant: Innovo Therapeutics Inc., Seoul 04174 (KR)
(72) Inventor: LIM, Dong Chul, Daejeon 35203 (KR); PARK, Jung Gyu, Daejeon 34119 (KR); CHOI, Sei Hyun, Daejeon 34022 (KR)
(74) Representative: Graf von Stosch Patentanwaltsgesellschaft mbH
(86) International application number: PCT/KR2022/015097
(87) International publication number: WO 2023/059121

(57) **Abstract**

The present invention provides a pharmaceutical composition for inhibiting HSP47 comprising a benzofuranyl hydroxyphenyl methanone derivative compound with a specific chemical structure, or a pharmaceutically acceptable salt thereof, and a method of preparation thereof.

The benzofuranyl hydroxyphenyl methanone derivative compound included in the pharmaceutical composition for inhibiting HSP47 of the present invention can be effectively used in the medical and pharmaceutical fields for inhibiting HSP47, such as preventing or treating fibrosis, inflammatory diseases, and cancer.

## Description

### FIELD

The present invention relates to novel benzofuranyl hydroxyphenyl methanone derivative compound, and more specifically, to a pharmaceutical composition for inhibiting HSP47 comprising a benzofuranyl hydroxyphenyl methanone derivative and a method for preparing the same.

### BACKGROUND

Heat shock protein 47 (HSP47) is a stress-induced endoplasmic reticulum protein that acts as a chaperone protein for the formation of the three-dimensional structure of collagen and extracellular secretion. HSP47 has been reported to be upregulated in fibrosis of various tissues such as cirrhosis, pulmonary fibrosis, keloids, hypertrophic scars and glomerulosclerosis. According to the literature, it has been reported that inhibiting the HSP47 protein inhibits collagen secretion in cells that produce collagen. It has also been reported that inhibition of HSP47 protein induces apoptosis of collagen-producing cells (Ito S, *et. al*., 2017). These results suggest that inhibition of HSP47 protein is an effective and specific therapeutic target for anti-fibrosis treatment.

Fibrosis is the abnormal accumulation of collagen following injury or inflammation that alters the structure and function of various tissues. Regardless of where fibrosis occurs, most etiologies of fibrosis involve an excessive accumulation of collagen that replaces normal tissue. Progressive fibrosis in the liver, lungs, or skin is a disease of high unmet medical need for which there is currently no specific treatment.

The function of HSP47 is not limited to collagen production, but has been reported to promote inflammation and angiogenesis by promoting the expression of MCP-1 (Monocyte chemoattractant protein-1), one of the inflammatory mediator proteins, in bladder cancer cells (Ma Wenlong *et. al*., 2021).

HSP47 has also been reported to be expressed in cancer cells, and it has been reported that increased expression of HSP47 facilitates the metastasis of many cancer cells, regardless of whether collagen is expressed or not, and increases mortality. It has also been reported that cancer progression is suppressed when HSP47 expression is suppressed through genetic methods (Parveen A *et. al*., 2020).

### [Prior art references]

### [Patent Literature]

EP 2165705 A1 (2010.03.24)
CN 102718735 B (2014.04.23)

### [Non-Patent Literature]

Benzofurans. XXXI. Relation of structure to uricosuric activity of certain p-hydroxybenzoylbenzofurans in the human By: Delbarre, Florian; Deltour, Guy; Rose, Alain; Olivier, J. L.; Binon, Fernand Chimica Therapeutica (1968), 3(6), 470-4 | Language: French, Database: CAplus.

Ito S et al., A small-molecule compound inhibits a collagen-specific molecular chaperone and could represent a potential remedy for fibrosis. (2017) JBC.

Thomson CA et al., Identification of Small Molecule Chemical Inhibitors of the Collagen-Specific Chaperone Hsp47. (2005) J. Med. Chem.

Ma Wenlong et. al., HSP47 contributes to angiogenesis by induction of CCL2 in bladder cancer Cell Signal. 2021 85:110044.

Parveen A et al., Mutational hotspots of HSP47 and its potential role in cancer and bone-disorders. Genomics. 2020 Jan;112(1):552-566.

### OBJECT OF THE INVENTION

The problem to be addressed by the present invention is to recognize that there is a close relationship between HSP47 and excessive accumulation of collagen, and to provide an HSP47 inhibitory substance that has therapeutic efficacy against diseases such as fibrosis and cancer by inhibiting the HSP47 protein.

Further, the problem to be addressed by the present invention is to provide a pharmaceutical composition for preventing or treating HSP47-related diseases, comprising the benzofuranyl hydroxyphenyl methanone derivative compound as an active ingredient.

Further, the problem to be addressed by the present invention is to provide a method for preventing or treating HSP47-related diseases, comprising: administering the benzofuranyl hydroxyphenyl methanone derivative compound, or a pharmaceutically acceptable salt thereof.

Further, the problem to be addressed by the present invention is to provide a use of the benzofuranyl hydroxyphenyl methanone derivative compound, or a pharmaceutically acceptable salt thereof in manufacture of a medicament for preventing or treating HSP47-related diseases.

Further, the problem to be addressed by the present invention is to provide a method for preparing the benzofuranyl hydroxyphenyl methanone derivative compound, or a pharmaceutically acceptable salt thereof.

The problem to be addressed by the present invention is not limited to the problems mentioned above, and other technical problems not mentioned can be clearly understood by those skilled in the art from the description below.

### SUMMARY

To address the problem above, according to one aspect of the present invention, there is provided a benzofuranyl hydroxyphenyl methanone derivative compound represented by the following Formula I, or a pharmaceutically acceptable salt thereof, wherein: 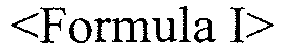 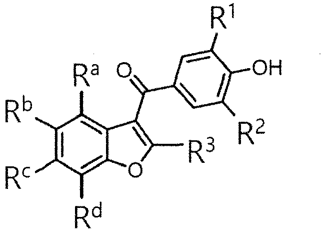
R¹ and R² are halogen,
R³ is hydrogen or C₁-C₃ alkyl,
R^{a}, R^{b} and R^{d} are independently hydrogen, C₁-C₃ alkyl or halogen,
R^{c} is hydrogen, C₁-C₃ alkyl, halogen or C₁-C₃ haloalkyl.

According to another aspect of the present invention, there is provided a pharmaceutical composition for preventing or treating HSP47-related diseases, comprising the benzofuranyl hydroxyphenyl methanone derivative compound represented by the above Formula I, or a pharmaceutically acceptable salt thereof as an active ingredient.

According to another aspect of the present invention, there is provided a method for preventing or treating HSP47-related diseases, comprising: administering the benzofuranyl hydroxyphenyl methanone derivative compound represented by the above Formula I, or a pharmaceutically acceptable salt thereof, to a subject in need thereof.

According to another aspect of the present invention, there is provided a use of the benzofuranyl hydroxyphenyl methanone derivative compound represented by the above Formula I, or a pharmaceutically acceptable salt thereof in manufacture of a medicament for preventing or treating HSP47-related diseases.

According to another aspect of the present invention, there is provided a method for preparing the benzofuranyl hydroxyphenyl methanone derivative compound represented by the above Formula I, or a pharmaceutically acceptable salt thereof, comprising: preparing a compound of Formula I-2 below by a nucleophilic substitution reaction from a compound of Formula I-1 below; reducing the compound of Formula 1-2 obtained above to prepare a compound of Formula 1-3 below; reacting the compound of Formula I-3 obtained above with 4-anisoyl chloride to prepare a compound of Formula I-4 below; removing the methyl of the methoxy of the compound of Formula I-4 obtained above to prepare a compound of Formula I-5 below having a hydroxy substituent; and obtaining a compound of Formula I below from the compound of Formula I-5 obtained above: 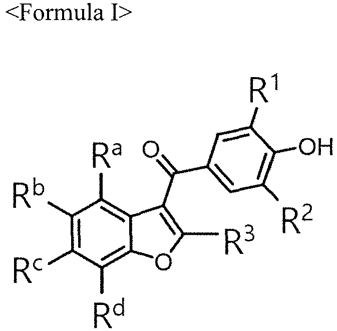 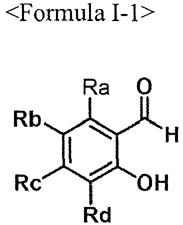 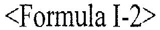 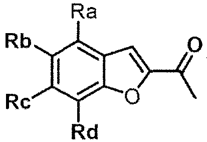 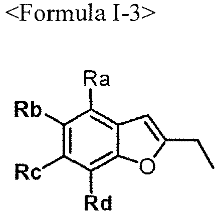 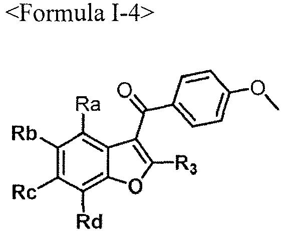 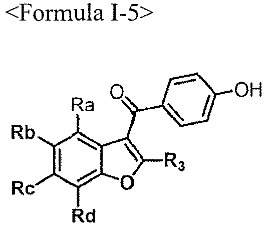 wherein in Formula I, Formula I-1, Formula 1-2, Formula I-3, Formula 1-4 and Formula I-5, R¹, R², R³, R^{a}, R^{b}, R^{c} and R^{d} are the same as defined above.

According to the present invention, it has been discovered that the benzofuranyl hydroxyphenyl methanone derivative compound represented by Formula I, can be used for HSP47 inhibition, such as the prevention or treatment of fibrosis and cancer, by suppressing collagen accumulation in liver, lung, kidney and skin tissues and killing cells that excessively produce collagen in a pathological state.

Therefore, the benzofuranyl hydroxyphenyl methanone derivative compound of the present invention can be effectively used in the medical and pharmaceutical fields for inhibiting HSP47, such as preventing or treating fibrosis and cancer.

The effects of the present invention are not limited to the above effects, but should be understood to include all effects that can be inferred from the description of the invention or the composition of the invention as recited in the patent claims.

### BRIEF DESCRIPTION OF THE DRAWINGS

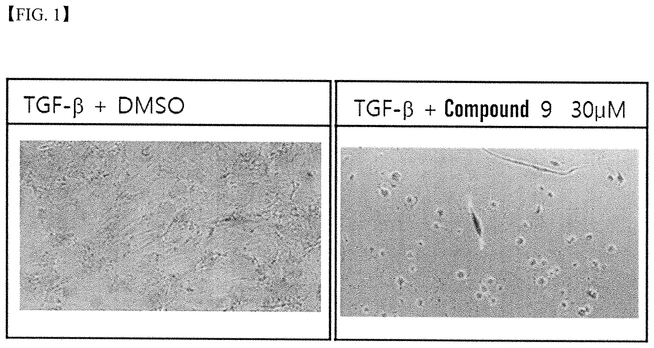

FIG. 1 shows the results of the test substance (compound 9) inhibiting fibrosis in KEL FIB skin fibroblasts.

### DETAILED DESCRIPTION OF THE INVENTION

The present invention provides a benzofuranyl hydroxyphenyl methanone derivative compound represented by the following Formula I, or a pharmaceutically acceptable salt thereof, wherein: 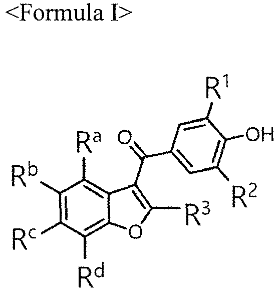
R¹ and R² are halogen,
R³ is hydrogen or C₁-C₃ alkyl,
R^{a}, R^{b} and R^{d} are independently hydrogen, C₁-C₃ alkyl or halogen,
R^{c} is hydrogen, C₁-C₃ alkyl, halogen or C₁-C₃ haloalkyl.

In one embodiment, R¹ and R² may be bromo or iodo.

In one embodiment, R³ may be a substituent selected from the group consisting of hydrogen, methyl and ethyl.

In one embodiment, R^{a} may be a substituent selected from the group consisting of hydrogen, methyl, fluoro and chloro.

In one embodiment, R^{b} may be a substituent selected from the group consisting of hydrogen, methyl, ethyl and bromo.

In one embodiment, R^{c} may be a substituent selected from the group consisting of hydrogen, methyl, ethyl, trifluoromethyl, chloro and bromo.

In one embodiment, R^{d} may be a substituent selected from the group consisting of hydrogen, methyl, ethyl, fluoro, chloro and bromo.

According to the present invention, representative examples of benzofuranyl hydroxyphenyl methanone derivative compounds represented by Formula I is selected from the group consisting of:
(4-chloro-2-ethylbenzofuran-3-yl)(3,5-dibromo-4-hydroxyphenyl)methanone,
(3,5-dibromo-4-hydroxyphenyl)(2-ethyl-5-methylbenzofuran-3-yl)methanone,
(3,5-dibromo-4-hydroxyphenyl)(2,6-diethylbenzofuran-3-yl)methanone,
(3,5-dibromo-4-hydroxyphenyl)(2-ethyl-7-methylbenzofuran-3-yl)methanone,
(7-chloro-2-ethylbenzofuran-3-yl)(3,5-dibromo-4-hydroxyphenyl)methanone,
(7-bromo-2-ethylbenzofuran-3-yl)(3,5-dibromo-4-hydroxyphenyl)methanone,
(3,5-dibromo-4-hydroxyphenyl)(2-ethyl-7-fluorobenzofuran-3-yl)methanone,
(2-ethyl-5-methylbenzofuran-3-yl)(4-hydroxy-3,5-diiodophenyl)methanone,
(5-bromo-2-ethylbenzofuran-3-yl)(4-hydroxy-3,5-diiodophenyl)methanone,
(2-ethyl-6-methylbenzofuran-3-yl)(4-hydroxy-3,5-diiodophenyl)methanone,
(6-bromo-2-ethylbenzofuran-3-yl)(4-hydroxy-3,5-diiodophenyl)methanone,
(6-chloro-2-ethylbenzofuran-3-yl)(3,5-dibromo-4-hydroxyphenyl)methanone,
(2-ethyl-7-methylbenzofuran-3-yl)(4-hydroxy-3,5-diiodophenyl)methanone,
(7-chloro-2-ethylbenzofuran-3-yl)(4-hydroxy-3,5-diiodophenyl)methanone,
(7-bromo-2-ethylbenzofuran-3-yl)(4-hydroxy-3,5-diiodophenyl)methanone,
benzofuran-3-yl(3,5-dibromo-4-hydroxyphenyl)methanone,
(6-bromo-2-methylbenzofuran-3-yl)(3,5-dibromo-4-hydroxyphenyl)methanone,
(5-bromo-2-methylbenzofuran-3-yl)(3,5-dibromo-4-hydroxyphenyl)methanone,
(6-chloro-2-methylbenzofuran-3-yl)(4-hydroxy-3,5-diiodophenyl)methanone,
(3,5-dibromo-4-hydroxyphenyl)(2,5-diethylbenzofuran-3-yl)methanone,
(3,5-dibromo-4-hydroxyphenyl)(2,7-diethylbenzofuran-3-yl)methanone,
(3,5-dibromo-4-hydroxyphenyl)(2-ethyl-6-(trifluoromethyl)benzofuran-3-yl)methanone,
(3,5-dibromo-4-hydroxyphenyl)(2-ethyl-4-methylbenzofuran-3-yl)methanone, and
(3,5-dibromo-4-hydroxyphenyl)(2-ethyl-4-fluorobenzofuran-3-yl)methanone.

This specification uses the following definitions when defining the compound represented by Formula I unless specifically defined.

The term "alkyl" refers to a straight or branched chain hydrocarbonyl group, preferably C₁-C₁₀ alkyl. Examples of alkyl include, but are not limited to, methyl, ethyl, n-propyl, *iso*propyl, n-butyl, iso-butyl, tert-butyl, n-pentyl, iso-pentyl, n-hexyl, 3-methylhexyl, 2,2-dimethylpentyl, 2,3-dimethylpentyl, n-heptyl, n-octyl, n-nonyl and n-decyl.

The term "halogen" or "halo" refers to fluorine (F), chlorine (Cl), bromine (Br) or iodine (I).

As used herein, "haloalkyl" refers to an alkyl group having one or more hydrogen atoms substituted by a halogen (e.g., mono-haloalkyl, di-haloalkyl and tri-haloalkyl). Such groups include, but are not limited to, chloromethyl, fluoromethyl, difluoromethyl, trifluoromethyl, 1-chloro-2-fluoromethyl and 2-fluoroisobutyl.

The compound represented by Formula I according to the present invention can be prepared and used in the form of prodrugs, hydrates, solvates and pharmaceutically acceptable salts to enhance *in vivo* absorption or increase solubility, so the prodrugs, hydrates, solvates and pharmaceutically acceptable salts are also within the scope of the present invention.

The term "prodrug" refers to a substance that is transformed *in vivo* into a parent drug. Prodrugs are often used because, in some cases, they are easier to administer than the parent drug. For example, they may be bioavailable by oral administration, whereas the parent drug may not be. Prodrugs may also have improved solubility in pharmaceutical compositions than the parent drug. For example, a prodrug may be an *in vivo* hydrolysable ester of the compound according to the present invention and a pharmaceutically acceptable salt thereof. Another example of a prodrug may be a short peptide (poly-amino acid) in which the peptide is coupled to an acid group that is metabolically converted to reveal the active site.

The term "hydrate" refers to a compound of the present invention or a salt thereof containing a stoichiometric or non-stoichiometric amount of water bound by non-covalent intermolecular forces.

The term "solvate" refers to a compound of the present invention or a salt thereof containing a stoichiometric or non-stoichiometric amount of solvent bound by non-covalent intermolecular forces. Preferred solvents therefor include solvents that are volatile, non-toxic, and/or suitable for administration to humans..

The term "isomer" refers to a compound of the present invention or a salt thereof that has the same chemical formula or molecular formula but is structurally or sterically different. Such isomers include both structural isomer such as tautomer, and stereoisomers such as R or S isomers with asymmetric carbon center and geometric isomers (trans, cis). All of these isomers and their mixtures thereof are also included within the scope of the present invention.

The term "pharmaceutically acceptable salt" refers to a salt form of a compound that does not cause serious irritation to the organism to which the compound is administered and does not impair the biological activity and physical properties of the compound. The pharmaceutical salts include an acid addition salt formed by an acid containing a pharmaceutically acceptable anion and forming a non-toxic acid addition salt, for example, inorganic acids such as hydrochloric acid, sulfuric acid, nitric acid, phosphoric acid, hydrobromic acid, hydrogen iodide, etc., organic carbon acids such as tartaric acid, formic acid, citric acid, acetic acid, trichloroacetic acid, trifluoroacetic acid, gluconic acid, benzoic acid, lactic acid, fumaric acid, malic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline and triethylamine etc. The compound of Formula I according to the present invention can also be converted into its salt by conventional methods.

The present invention also provides a method for preparing the compound represented by Formula I above, comprising:
(1) Preparing a compound of Formula I-2 below by a nucleophilic substitution reaction from a compound of Formula I-1 below

This step is to react the compound of Formula I-1 below with chloroacetone in the presence of potassium carbonate to synthesize a benzofuran ring compound. The solvent used can be any solvent commonly used in nucleophilic substitution reactions, such as acetone, tetrahydrofuran, N,N-dimethylformamide, and the like, and the compound of Formula I-2 can be obtained by reacting at a suitable temperature (e.g., 70 °C) for a suitable reaction time (e.g., about 6 hours).

(2) Reducing the compound of Formula I-2 obtained above to prepare a compound of Formula I-3 below

This step is a reaction in which the ketone of the compound of Formula I-2 obtained above is reduced using hydrazine and potassium hydroxide. The compound of Formula I-2 obtained above is dissolved in diethylene glycol, hydrazine is added, and potassium hydroxide is slowly added at a high temperature (e.g., 150 °C) and reacted for an appropriate reaction time (e.g., 1 hour) to obtain the compound of Formula I-3.

(3) Reacting the compound of Formula I-3 obtained above with 4-anisoyl chloride to prepare a compound of Formula I-4 below

This step is a Friedel-Crafts acylation reaction in which the benzofuran compound of Formula I-3 obtained above is reacted with 4-anisoyl chloride (4-methoxybenzoyl chloride) and tin tetrachloride (SnCl₄), a Lewis acid, to obtain the compound of Formula I-4 below. The solvent used can be any solvent conventionally used in Friedelkrafft acylation reactions, e.g., dichloromethane, carbon disulfide.

(4) Removing the methyl of the methoxy of the compound of Formula I-4 obtained above to prepare a compound of Formula I-5 below having a hydroxy substituent

This step can be either 1) a demethylation reaction using sodium ethanolate, or 2) a demethylation reaction using boron tribromide. The reaction using sodium ethanethiolate in method 1) can be carried out primarily in a solvent such as, but not limited to, N,N-dimethylformamide. The demethylation reaction using boron tribromide in the second method 2) can be carried out primarily in dichloromethane solvent, but is not limited to.

(5) Obtaining a compound of Formula I below from the compound of Formula I-5 obtained above

This step is a reaction to introduce two bromo groups or iodo groups into the compound of Formula I-5 obtained above. 1) The reaction to introduce a bromo group is carried out by adding bromine or N-bromosuccinimide to the compound of Formula I-5 below. The solvent used in this reaction is a solvent conventionally used in bromination reactions, such as dichloromethane or acetone. 2) The reaction to introduce an iodo group can be obtained by reacting the compound of Formula I-5 below with iodo and silver nitrate. The solvent used in this reaction may be, but is not limited to, an alcoholic solvent such as ethanol. 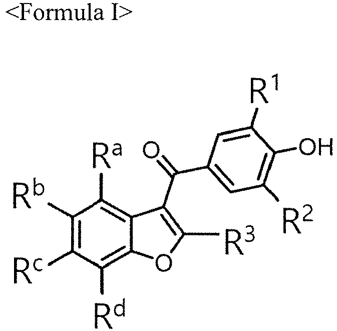 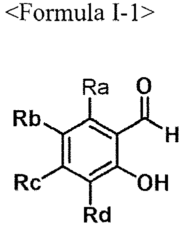 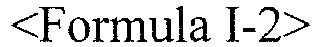 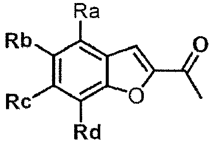 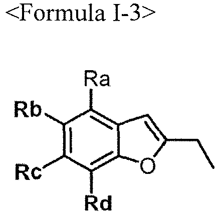 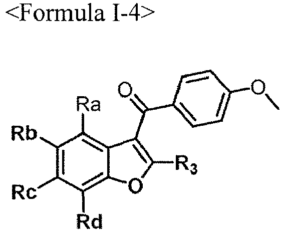 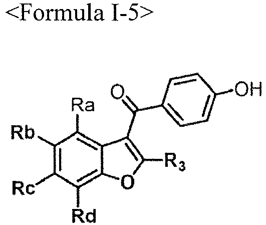 wherein in Formula I, Formula 1-1, Formula I-2, Formula I-3, Formula I-4 and Formula I-5, R¹, R², R³, R^{a}, R^{b}, R^{c} and R^{d} are the same as defined above.

Schemes 1 to 24 of the Examples are exemplified as methods for preparing the compound represented by Formula I of the present invention, and the preparation methods of Schemes 1 to 24 do not limit the method of preparing the compound represented by Formula I according to the present invention. It will be appreciated that the preparation methods of Schemes 1 to 24 are exemplary only and can be readily modified by one of ordinary skill in the art depending on specific substituents.

The present invention also provides a pharmaceutical composition for inhibiting HSP47 comprising the benzofuranyl hydroxyphenyl methanone derivative compound represented by the above Formula I, or a pharmaceutically acceptable salt thereof as an active ingredient.

The present invention also provides a method for preventing or treating related diseases for the purpose of inhibiting HSP47, comprising: administering a therapeutically effective amount of the benzofuranyl hydroxyphenyl methanone derivative compound represented by the above Formula I, or a pharmaceutically acceptable salt thereof, to a subject in need thereof.

The present invention also provides a use of the benzofuranyl hydroxyphenyl methanone derivative compound represented by the above Formula I, or a pharmaceutically acceptable salt thereof in manufacture of a medicament for the purpose of inhibiting HSP47.

In one embodiment, said use for inhibiting HSP47 may be for the prevention or treatment of fibrosis, which may be achieved by inhibiting HSP47. In this case, said fibrosis may be one or more selected from the group consisting of liver cirrhosis, pulmonary fibrosis, keloid, hypertrophic scar and renal fibrosis (glomerulosclerosis).

In one embodiment, said use for inhibiting HSP47 may be for the prevention or treatment of cancer, which may be achieved by inhibiting HSP47. In this case, said cancer is one or more selected from the group consisting of breast cancer, colon cancer and cancer-related fibrosis.

In one embodiment, said use for inhibiting HSP47 may be for the treatment of an inflammatory disease through inhibition of pro-inflammatory secretion, which may be achieved by inhibiting HSP47. In this case, the inflammatory disease may be an auto inflammatory disease and an autoimmune disease associated with an increase in MCP-1.

In one embodiment, the inflammatory disease is preferably, but not limited to, one or more selected from the group consisting of liver cancer, prostate cancer, melanoma, ovarian cancer, neuroinflammatory diseases, arteriosclerosis, organ transplantation, dermatitis, atopic dermatitis, asthma, conjunctivitis, periodontitis, rhinitis, otitis media, pharyngitis, tonsillitis, pneumonia, stomach ulcer, pancreatitis, gastritis, psoriasis, nephropathy, neuroinflammatory diseases, myasthenia gravis, Crohn's disease, inflammatory bowel disease, colitis, gout, ankylosing spondylitis, lupus, fibromyalgia, psoriasis, rheumatoid arthritis, osteoarthritis, osteoporosis, hepatitis, cystitis, nephritis, Sjögren's syndrome and multiple sclerosis.

As a result of evaluating whether the compounds of the present invention inhibit HSP47 activity, it was confirmed that the EC₅₀ for HSP47 was 15.89 ~ 104.5 µM, or that the compounds showed HSP47 inhibitory activity at a concentration of 100 µM. Regarding the inhibition of fibrosis in tissue cells, the compounds showed anti-fibrosis activity in lung epithelial cells and hepatic stellate cells, and were shown to inhibit fibrosis in skin fibroblasts. In addition, with respect to disease manifestations of fibrosis in an animal model of induced liver fibrosis, the above compounds reduced the level of hyaluronic acid in the blood, which is a biomarker of liver fibrosis, and reduced the content of 4-hydroxyproline in liver tissue, thereby reducing fibrosis-induced collagen deposition. Furthermore, the above compounds were shown to significantly reduce the area of liver fibrosis by inhibiting fibrosis of liver tissue through histological staining.

As a result of evaluating whether the compounds of the present invention inhibit MCP-1 secretion, the EC₅₀ for MCP-1 was 1.374 ~ 17.14 µM. Regarding the inhibition of MCP-1 secretion from tissue cells, the above compounds were shown to inhibit MCP-1 secretion from hepatic stellate cells.

In one embodiment, the pharmaceutical composition may further comprise a pharmaceutically acceptable diluent or carrier.

Specifically, the pharmaceutical compositions of the present invention may comprise a pharmaceutically acceptable carrier, each of which may be formulated according to conventional methods in the form of oral formulations such as powders, granules, tablets, capsules, suspensions, emulsions, syrups, aerosols; topicals; suppositories; and sterile injectable solutions. The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate and mineral oil, and the like. They also include diluents or excipients such as fillers, bulking agents, binders, wetting agents, disintegrating agents, and surfactants. Oral solid dosage forms include tablets, pills, powders, granules, capsules, and the like, which may include at least one excipient, such as starch, calcium carbonate, sucrose, lactose, gelatin, and the like, and may include lubricants such as magnesium stearate and talc. Oral liquid preparations may include suspensions, oral solutions, emulsions, syrups, and the like, and may include diluents such as water and liquid paraffin, wetting agents, sweeteners, flavourings, preservatives, and the like. Parenteral preparations include sterilized aqueous solutions, non-aqueous solvents, suspensions, emulsions, creams, lyophilised preparations, and suppositories; non-aqueous solvents and suspensions include propylene glycol, polyethylene glycol, vegetable oils such as olive oil, and injectable esters such as ethylolate. Substrates for suppositories may be witepsol, macrogol, tween 61, cacao gum, laurin gum, glycerogelatin, etc.

The dosage of the active ingredient in the pharmaceutical composition of the present invention depends on the condition and weight of the patient, the extent of the disease, the formulation of the active ingredient, the route and duration of administration, and may be appropriately adjusted depending on the patient. For example, the compound of Formula I can be administered at a dose of 0.0001 to 1000 mg/kg per day, preferably 0.01 to 1000 mg/kg, and the dose may be administered once or in several divided doses per day. Furthermore, the pharmaceutical composition of the present invention may comprise the compound of Formula I from 0.001 to 90% by weight, based on the total weight of the composition.

The pharmaceutical composition of the present invention may be administered to mammals such as rats, mice, livestock, and humans by various routes, for example, orally, by dermal, intraperitoneally, rectally or intravenously, intramuscular, subcutaneous, intrauterine dura, or intracerebroventricular injection.

The additive may include a pharmaceutically acceptable carrier or diluent, each of which may be formulated according to conventional methods in the form of oral formulations such as powders, granules, tablets, capsules, suspensions, emulsions, syrups, aerosols; topicals; suppositories; and sterile injectable solutions. The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate and mineral oil, and the like. They also include diluents or excipients such as fillers, bulking agents, binders, wetting agents, disintegrating agents, and surfactants. Oral solid dosage forms include tablets, pills, powders, granules, capsules, and the like, which may include at least one excipient, such as starch, calcium carbonate, sucrose, lactose, gelatin, and the like, and may include lubricants such as magnesium stearate and talc. Oral liquid preparations may include suspensions, oral solutions, emulsions, syrups, and the like, and may include diluents such as water and liquid paraffin, wetting agents, sweeteners, flavourings, preservatives, and the like. Parenteral preparations include sterilized aqueous solutions, non-aqueous solvents, suspensions, emulsions, creams, lyophilised preparations, and suppositories; non-aqueous solvents and suspensions include propylene glycol, polyethylene glycol, vegetable oils such as olive oil, and injectable esters such as ethylolate. Substrates for suppositories may be witepsol, macrogol, tween 61, cacao gum, laurin gum, glycerogelatin, etc.

The pharmaceutical compositions of the present invention can be administered to mammals, such as livestock and humans, by various routes, e.g., by oral, dermal, subcutaneous, intramuscular, intravenous, intraperitoneal, rectal, intrauterine, intrathecal or cerebrovascular injection, and topical administration. Accordingly, the compositions of the present invention can be formulated in various forms, such as tablets, capsules, aqueous solutions or suspensions. For oral tablets, a carrier such as lactose, cornstarch, or the like, and a disintegrating agent such as magnesium stearate may be typically added. For oral capsules, lactose and/or dry cornstarch may be used as diluents. If an oral aqueous suspension is desired, the active ingredient may be combined with an emulsifier and/or suspending agent. If necessary, specific sweeteners and/or flavoring agents may be added. For intramuscular, intraperitoneal, subcutaneous and intravenous administration, a sterile solution of the active ingredient is usually prepared, and the pH of the solution should be adjusted and buffered accordingly. For intravenous administration, the total concentration of the solute should be adjusted to impart isotonicity to the preparation. The composition according to the invention may be in the form of an aqueous solution containing a pharmaceutically acceptable carrier, such as brine with a pH of 7.4. The solution may be introduced into the intramuscular bloodstream of a patient by local injection.

The dosage of the active ingredient in the pharmaceutical composition of the present invention depends on the condition and weight of the patient, the extent of the disease, the formulation of the active ingredient, the route and duration of administration, and may be appropriately adjusted depending on the patient. For example, the active ingredient can be administered at a dose of 0.0001 to 1000 mg/kg per day, preferably 0.01 to 100 mg/kg, and the dose may be administered once or in several divided doses per day. Furthermore, the pharmaceutical composition of the present invention may comprise the active ingredient from 0.001 to 90% by weight, based on the total weight of the composition.

Hereinafter, the present invention will be described in more detail with Examples. However, the following Examples are intended to illustrate the present invention and are not intended to limit the scope of the present invention.

### Example 1. (4-chloro-2-ethylbenzofuran-3-yl)(3,5-dibromo-4-hydroxyphenyl)methanone

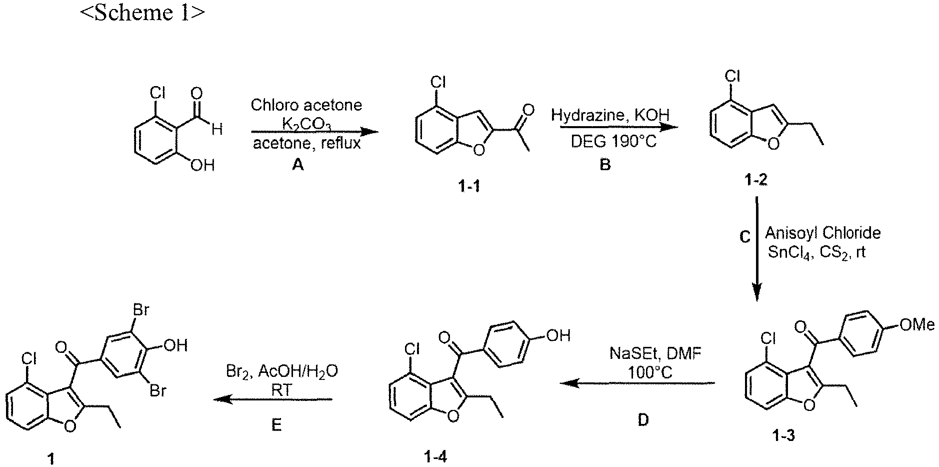

**Step A:** To a reaction flask containing 2-hydroxy-4-chlorobenzaldehyde (2 g), potassium carbonate (1.5 equivalents), and anhydrous acetone (20 mL), 1.2 equivalents of chloroacetone was added dropwise for 10 minutes, and the reaction solution was stirred at reflux for 3 hours. After completion of the reaction, the solid was removed by filtration, and the filtered liquid was dried over anhydrous magnesium sulfate, filtered, concentrated, and separated by column chromatography to give 2-acetylbenzofuran **(1-1)** (1.99 g, 78% yield).

**Step B:** To compound **1-1** (1.99 g, 0.011 mol) was added 6 equivalents of 80% hydrazine (monohydrate) and diethylene glycol (48 mL), stirred at 150 °C for 30 min, then 3 equivalents of potassium hydroxide was added slowly over 10 min. The reaction solution was stirred at reflux for 3 h, cooled to room temperature, diluted with water (100 mL), and extracted with ethyl acetate (120 mL × 3). The ethyl acetate layer was washed with brine (100 mL), dried over anhydrous sodium sulfate, filtered, the filtrate was concentrated and separated by column chromatography to give the target compound (**1-2,** 1.3 g, 72%).

**Step C:** Compound **1-2** (1.3 g, 0.0081 mol) was dissolved in anhydrous CS2 (20 vol) and cooled to 0°C, then 4-methoxybenzoyl chloride (1.5 equivalents) was added and stirred for 5 min. Tin (IV) chloride (1.5 equivalents) was slowly added dropwise to this reaction solution over 10 min, stirred at 0°C for 2 hours, and then stirred at room temperature for 22 h. After completion of the reaction, the reaction solution was diluted with water (200 mL) and extracted with dichloromethane (90 mL × 2). The extracted organic layer was washed sequentially with 1N hydrochloric acid solution (50 mL), 1N aqueous sodium hydroxide solution (50 mL), and brine (50 mL), dried over anhydrous sodium sulfate, filtered, and concentrated. The target compound (1-3, 1.98 g, 83%) was obtained by separation by chromatography.

**Step D:** Compounds **1-3** (1.98 g, 0.0067 mol) were dissolved in N,N-dimethylformamide (20 mL), followed by the addition of 2.5 equivalents of sodium ethanethiolate (NaSEt) at room temperature, and the reaction solution was stirred at 100 °C for 2 hours. After completion of the reaction, an aqueous solution of ammonium chloride (100 mL) was slowly added to dilute it, then extracted with dichloromethane (100 mL × 2), the extract was washed with brine (80 mL), the organic layer was dried over anhydrous sodium sulfate, filtered, concentrated and separated by column chromatography to give the target compound (**1-4,** 1.68 g, 89%).

**Step E:** Compounds **1-4** (100 mg, 0.35 mmol) were dissolved in glacial acetic acid (6 mL) and water (2 mL), followed by the addition of 2.2 equivalents of bromine diluted with glacial acetic acid (1.5 mL) and water (0.5 mL) over 15 min, and the reaction was stirred at room temperature for 20 hours. After completion of the reaction, the reaction solution was concentrated and separated by column chromatography to give the target compound (1, 86 mg, 55% yield).

¹H NMR (DMSO-d₆, 400 MHz) δ 7.90 (s, 3H), 7.65 (d, *J=* 7.9 Hz, 1H), 7.40 -7.26 (m, 2H), 2.75 - 2.58 (m, 2H), 1.17 (t, *J* = 7.5 Hz, 3H).

MS (ESI, m/z): Exact mass calculated [M+H]⁺: 456.88 ; found: 456.9.

### Example 2. (3,5-dibromo-4-hydroxyphenyl)(2-ethyl-5-methylbenzofuran-3-yl)methanone

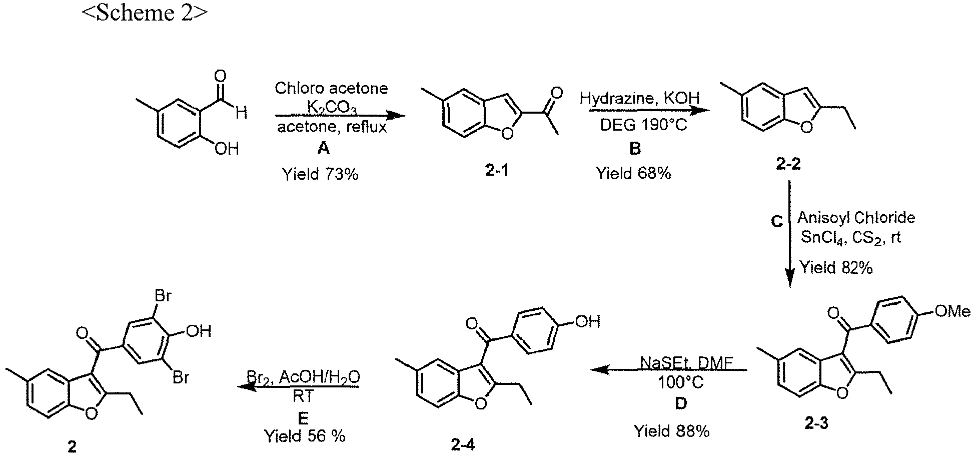

**Step A - Step E:** Compound **2** was obtained in a similar process to steps A-E of

### Example 1.

¹H NMR (DMSO-d₆, 400 MHz) δ 7.80 (s, 2H), 7.45 (d, *J=* 8.2 Hz, 1H), 7.33 - 6.99 (m, 2H), 2.71 (d, *J=* 7.4 Hz, 2H), 2.30 (s, 3H), 1.26 -1.09 (m, 3H).

MS (ESI, m/z): Exact mass calculated [M+H]⁺: 436.93; found: 437.1.

### Example 3. (3,5-dibromo-4-hydroxyphenyl)(2,6-diethylbenzofuran-3-yl)methanone

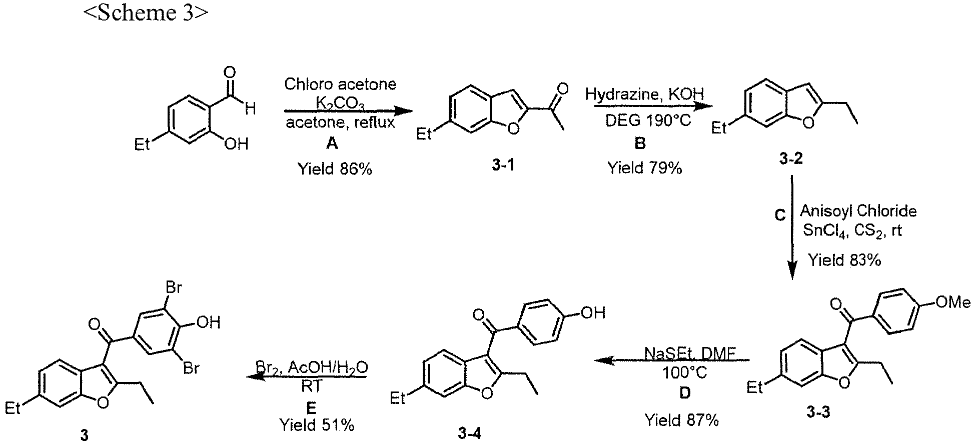

**Step A - Step E:** Compound 3 was obtained in a similar process to steps A-E of Example 1.

¹H NMR (DMSO-d₆, 400 MHz) δ 7.88 (s, 2H), 7.46 (s, 1H), 7.26 (d, *J=* 8.1 Hz, 1H), 7.10 (dd, *J=* 8.0, 1.4 Hz, 1H), 2.78 - 2.72 (m, 2H), 2.68 (q, *J=* 7.6 Hz, 2H), 1.23 - 1.17 (m, 6H).

### Example 4. (3,5-dibromo-4-hydroxyphenyl)(2-ethyl-7-methylbenzofuran-3-yl)methanone

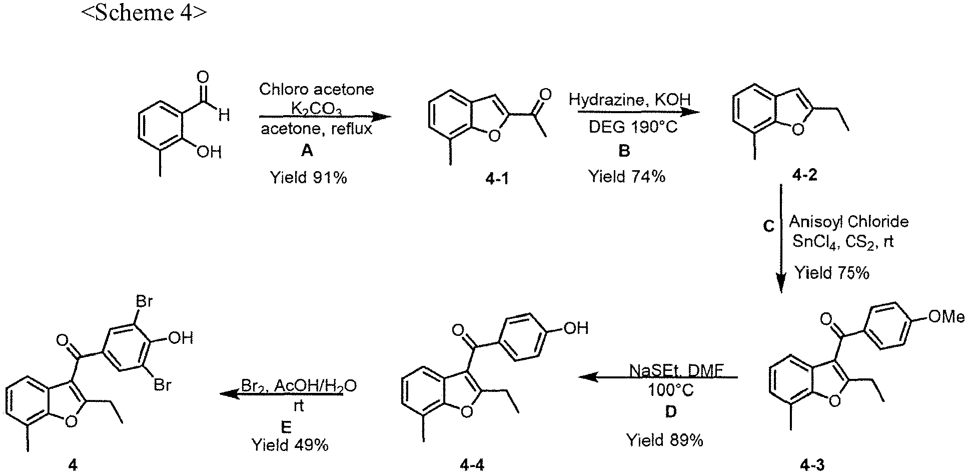

**Step A - Step E:** Compound 4 was obtained in a similar process to steps A-E of Example 1.

¹H NMR (DMSO-d₆, 400 MHz) 7.97 (s, 2H), 7.19 (dd, *J* = 7.4, 1.6 Hz, 1H), 7.14 -7.05 (m, 2H), 6.30 (s, 1H), 2.91 (q, *J=* 7.6 Hz, 2H), 2.54 (s, 3H), 1.36 (t, *J=* 7.5 Hz, 3H).

### Example 5. (7-chloro-2-ethylbenzofuran-3-yl)(3,5-dibromo-4-hydroxyphenyl)methanone

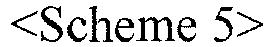

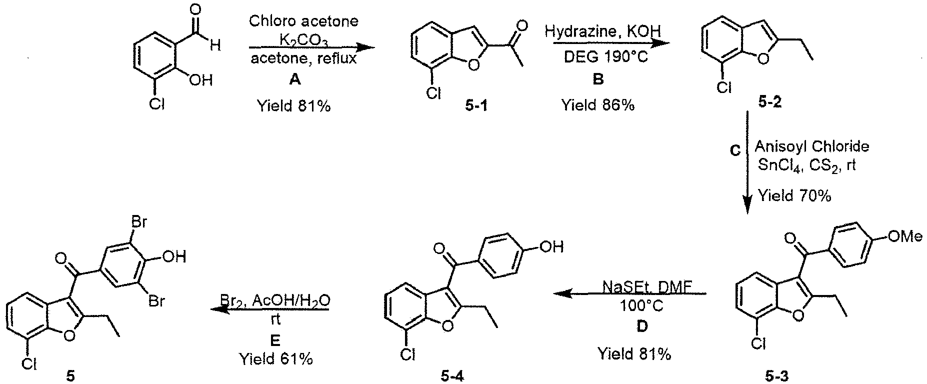

**Step A - Step E:** Compound **5** was obtained in a similar process to steps A-E of Example 1.

¹H NMR (DMSO-d₆, 400 MHz) δ 7.92 (s, 2H), 7.42 (dd, *J* = 7.8, 0.9 Hz, 1H), 7.36 (dd, *J* = 7.9, 1.0 Hz, 1H), 7.25 (t, *J* = 7.8 Hz, 1H), 2.78 (q, *J* = 7.5 Hz, 2H), 1.23 (t, *J* = 7.5 Hz, 3H).

### Example 6. (7-bromo-2-ethylbenzofuran-3-yl)(3,5-dibromo-4-hydroxyphenyl)methanone

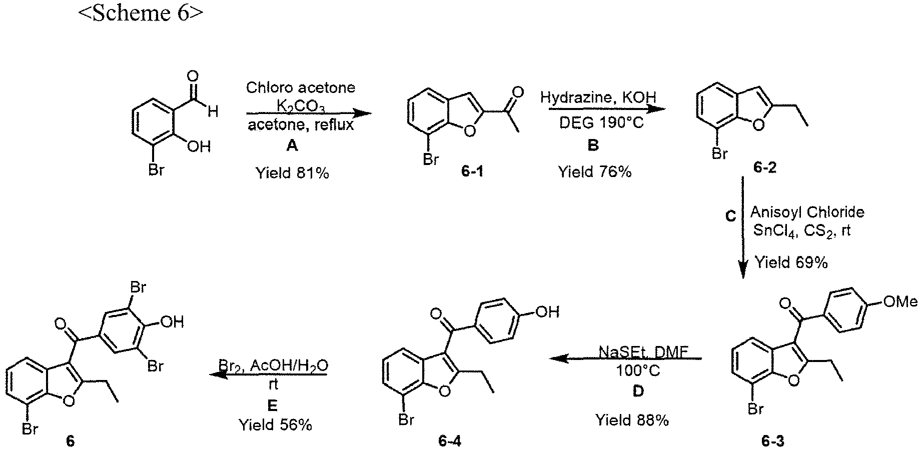

**Step A - Step E:** Compound 6 was obtained in a similar process to steps A-E of Example 1.

¹H NMR (DMSO-d₆, 400 MHz) 7.90 (s, 2H), 7.62 (d, *J=* 8.7 Hz, 1H), 7.58 (d, *J=* 2.0 Hz, 1H), 7.47 (dd, *J* = 8.7, 2.1 Hz, 1H), 2.73 (q, *J* = 7.5 Hz, 2H), 1.21 (t, *J* = 7.5 Hz, 3H).

### Example 7. (3,5-dibromo-4-hydroxyphenyl)(2-ethyl-7-fluorobenzofuran-3-yl)methanone

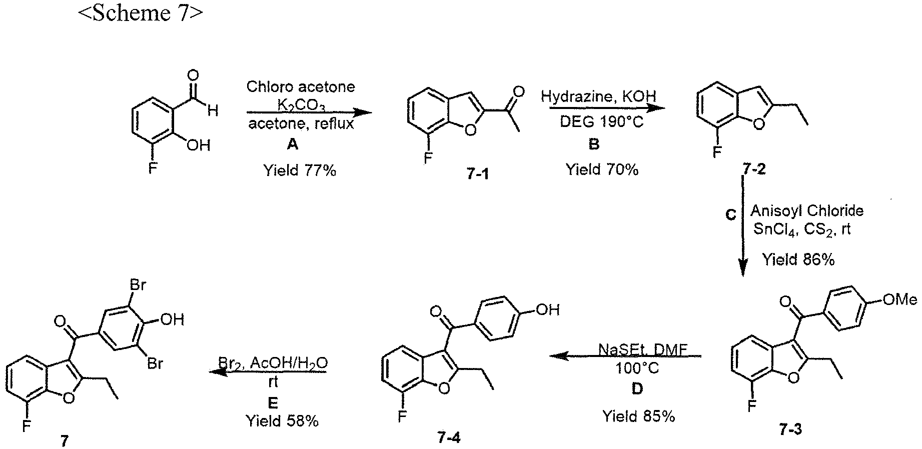

**Step A - Step E:** Compound 7 was obtained in a similar process to steps A-E of Example 1.

¹H NMR (DMSO-d6, 400 MHz) δ 7.93 (s, 2H), 7.59 (dd, *J=* 7.0, 6.2 Hz, 2H), 7.53 - 7.47 (m, 1H), 2.77 (q, *J=* 7.5 Hz, 2H), 1.22 (t, *J=* 7.4 Hz, 3H).

### Example 8. (2-ethyl-5-methylbenzofuran-3-yl)(4-hydroxy-3,5-diiodophenyl)methanone

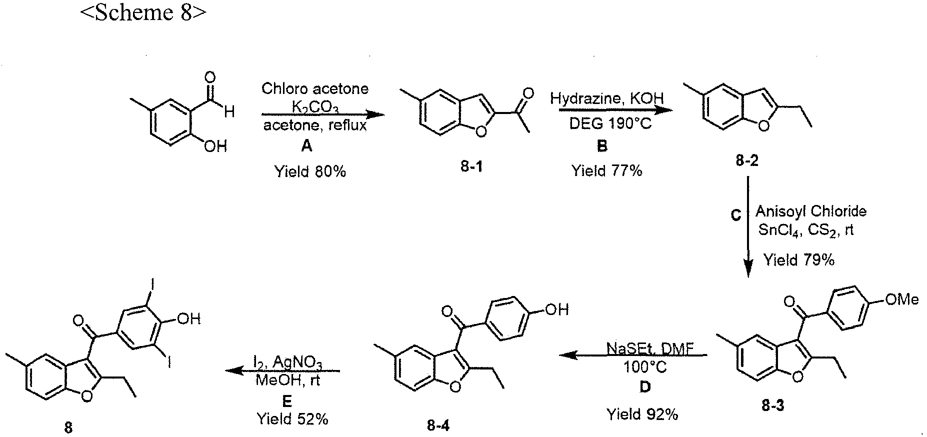

**Step A - Step D:** Compound **8-4** was obtained in a similar process to steps A-D of Example 1.

**Step E:** Compound **8-4** (100 mg, 0.35 mmol) was dissolved in methanol (3 mL), silver nitrate (2.5 equivalents) was added, iodine (2.5 equivalents) was added, and the mixture was stirred at room temperature for 20 hours. After completion of the reaction, the solid was filtered, the filtrate was concentrated and separated by column chromatography to give the target compound (**8**, 121 mg, 64% yield).

¹H NMR (DMSO-d₆, 400 MHz) δ 8.07 (s, 2H), 7.48 (d, *J=* 8.4 Hz, 1H), 7.26 - 7.19 (m, 1H), 7.12 (dd, J= 8.3, 1.6 Hz, 1H), 2.71 (q, *J* = 7.6 Hz, 2H), 1.21 (t, *J* = 7.5 Hz ,3H).

### Example 9. (5-bromo-2-ethylbenzofuran-3-yl)(4-hydroxy-3,5-diiodophenyl)methanone

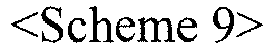

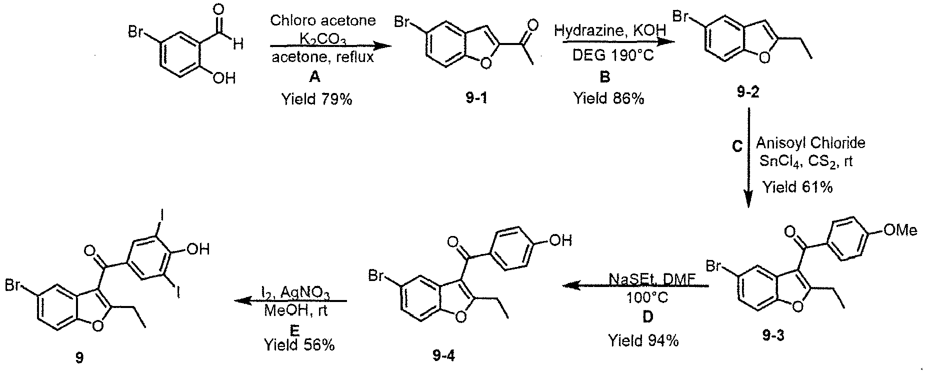

**Step A - Step E:** Compound 9 was obtained in a similar process to steps A-E of Example 8.

¹H NMR (DMSO-d6, 400 MHz) δ 8.15 (s, 2H), 7.64 (d, *J* = 1.6 Hz, 1H), 7.43 - 7.35 (m, 2H), 2.83 (dt, *J=* 7.7, 6.6 Hz, 2H), 1.34 (td, *J=* 7.5, 1.9 Hz, 3H).

### Example 10. (2-ethyl-6-methylbenzofuran-3-yl)(4-hydroxy-3,5-diiodophenyl)methanone

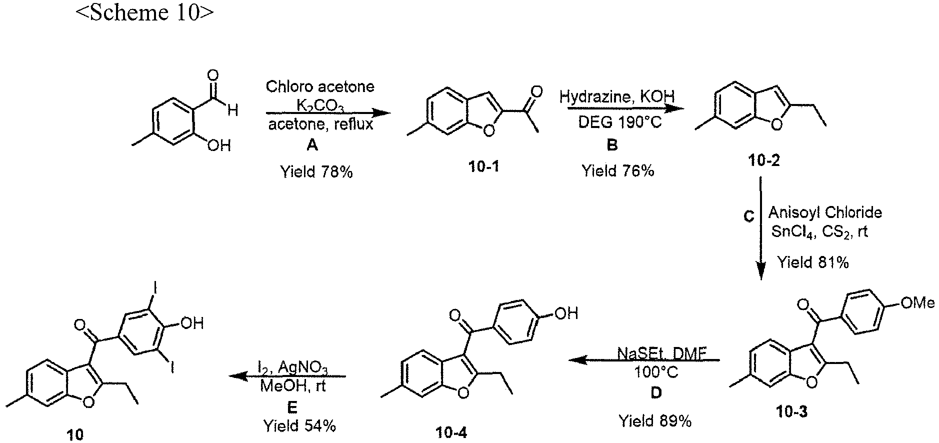

**Step** A - Step E: Compound 10 was obtained in a similar process to steps A-E of **Example 8.**

¹H NMR (DMSO-d₆, 400 MHz) δ.

Exact mass calculated [M+H]⁺ : 532.9 ; found: 532.9.

### Example 11. (6-bromo-2-ethylbenzofuran-3-yl)(4-hydroxy-3,5-diiodophenyl)methanone

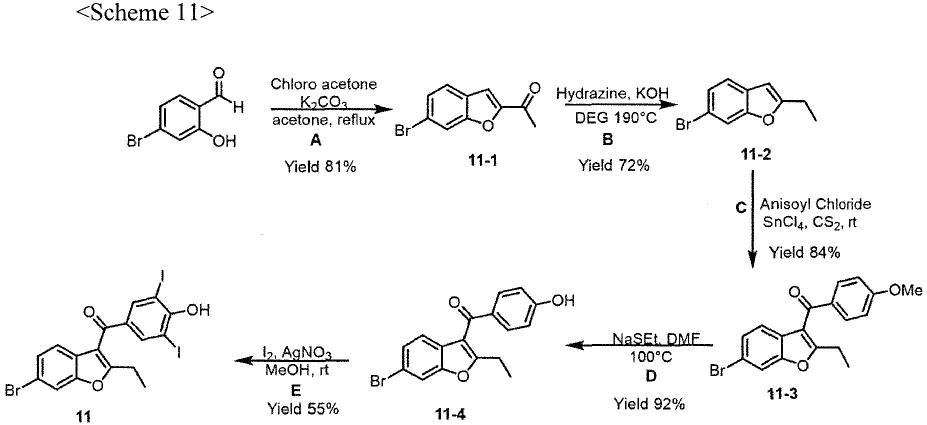

**Step A - Step E:** Compound **11** was obtained in a similar process to steps A-E of Example 8.

¹H NMR (DMSO-d₆, 400 MHz) δ 8.07 (s, 2H), 7.95 (d, *J=* 1.6 Hz, 1H), 7.43 (dd, *J=* 8.4, 1.7 Hz, 1H), 7.34 (d, *J* = 8.4 Hz, 1H), 2.73 (q, *J* = 7.5 Hz, 2H), 1.22 (t, *J* = 7.5 Hz, 3H).

### Example 12. (6-chloro-2-ethylbenzofuran-3-yl)(3,5-dibromo-4-hydroxyphenyl)methanone

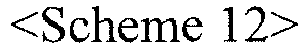

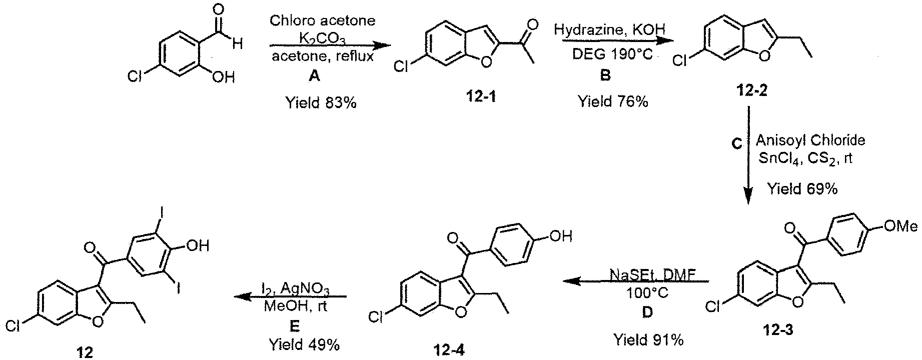

**Step A - Step E:** Compound 12 was obtained in a similar process to steps A-E of Example 8.

¹H NMR (DMSO-d₆, 400 MHz) δ 8.15 (s, 2H), 7.50 (d, *J* = 1.8 Hz, 1H), 7.35 (d, *J=* 8.5 Hz, 1H), 7.22 (dd, *J* = 8.5, 1.8 Hz, 1H), 2.86 (q, *J=* 7.6 Hz, 2H), 1.34 (t, *J=* 7.5 Hz, 3H).

### Example 13. (2-ethyl-7-methylbenzofuran-3-yl)(4-hydroxy-3,5-diiodophenyl)methanone

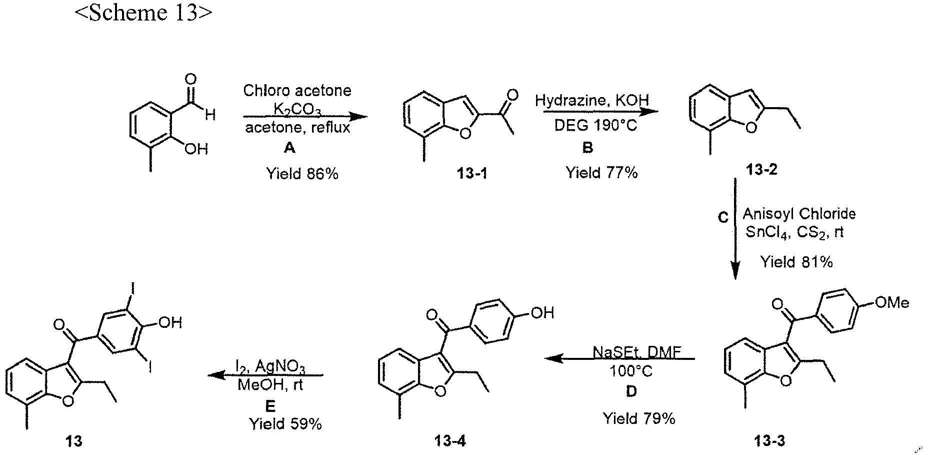

**Step A - Step E:** Compound 13 was obtained in a similar process to steps A-E of Example 8.

¹H NMR (DMSO-d₆, 400 MHz) δ 8.17 (s, 2H), 7.21 (d, *J=* 7.4 Hz, 1H), 7.15 - 7.05 (m,2H), 6.16 (s, 1H), 2.94 - 2.85 (m, 2H), 2.54 (s, 3H), 1.36 (t, *J=* 7.5 Hz, 3H).

### Example 14. (7-chloro-2-ethyl-3-yl)(4-hydroxy-3,5-diiodophenyl)methanone

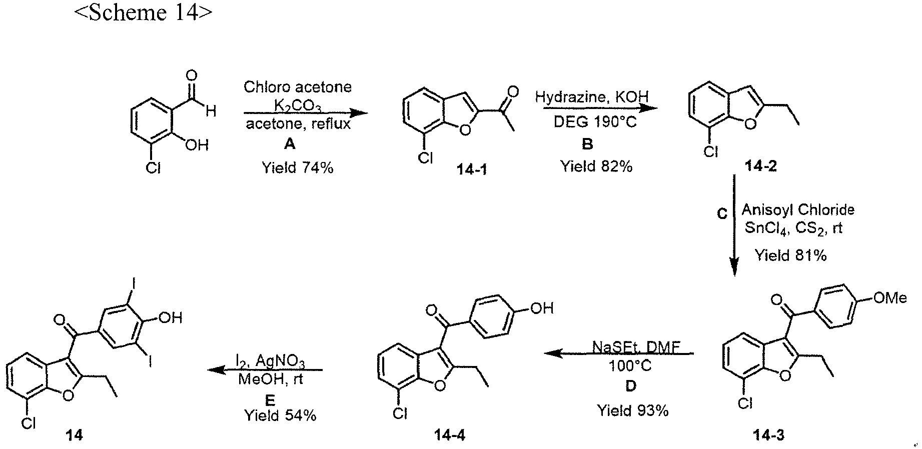

**Step A - Step E:** Compound **14** was obtained in a similar process to steps A-E of Example 8.

¹H NMR (DMSO-d₆, 400 MHz) δ 8.15 (s, 2H), 7.77 - 6.99 (m, 3H), 3.21 - 2.69 (m, 2H), 0.85 (d, *J* = 10.1 Hz, 3H).

### Example 15. (7-bromo-2-ethylbenzofuran-3-yl)(4-hydroxy-3,5-diiodophenyl)methanone

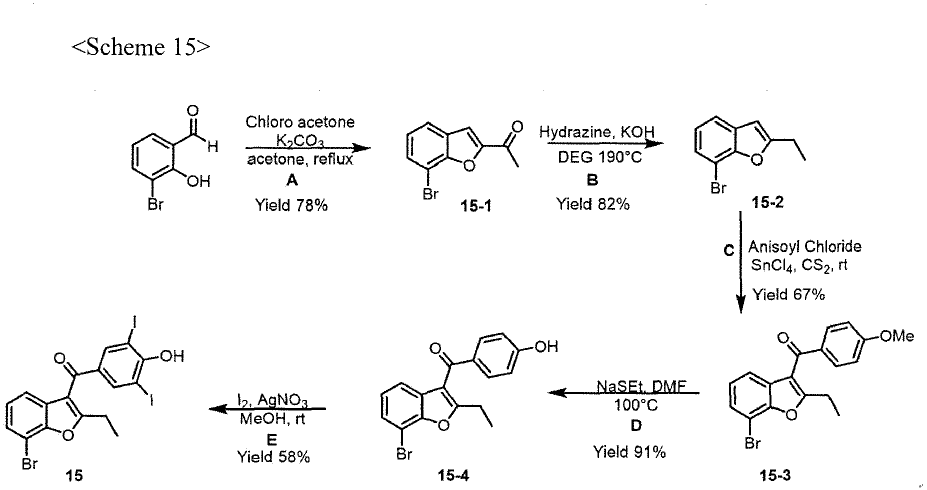

**Step A - Step E:** Compound **15** was obtained in a similar process to steps A-E of Example 8.

¹H NMR (DMSO-d₆, 400 MHz) δ 8.15 (s, 2H), 7.45 (d, *J=* 8.3 Hz, 1H), 7.44 - 7.31 (m, 1H), 7.17 - 7.04 (m, 1H), 3.15 - 2.79 (m, 2H), 1.60 (m, 3H).

### Example 16. benzofuran-3-yl(3,5-dibromo-4-hydroxyphenyl)methanone

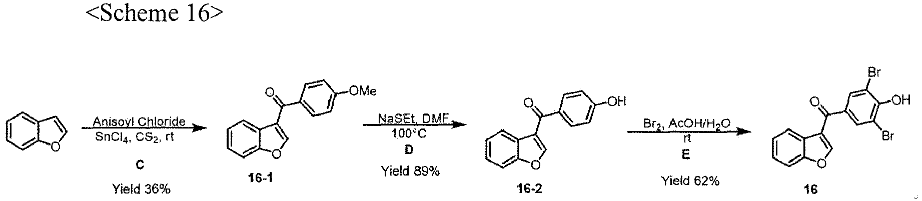

**Step C - Step E:** Compound **16** was obtained in a similar process to steps C-E of Example 8.

¹H NMR (DMSO-d₆, 400 MHz) 8.12 (s, 2H), 7.84 (d, *J=* 7.7 Hz, 1H), 7.80 (d, *J=* 0.7 Hz, 1H), 7.77 - 7.71 (m, 1H), 7.53 (ddd, *J* = 8.5, 7.2, 1.2 Hz, 1H), 7.38 - 7.31 (m, 1H).

### Example 17. (6-bromo-2-methylbenzofuran-3-yl)(3,5-dibromo-4-hydroxyphenyl)methanone

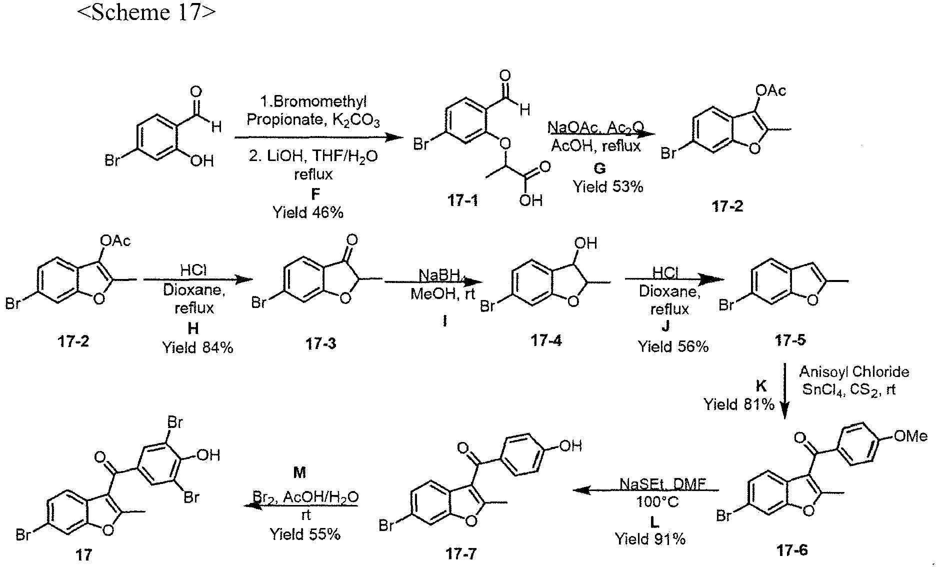

**Step F:** After dissolving 4-bromo salicylaldehyde (5 g, 32 mmol) in anhydrous acetonitrile (50 mL), 1.5 equivalents of potassium carbonate (1.5 equivalents) was added, and then 1.5 equivalents of methyl 2-bromopropionic acid was added dropwise for 10 minutes. The reaction solution was stirred at room temperature for 6 hours, the resulting solid was filtered, the filtrate was concentrated, lithium hydroxide aqueous solution (1N, 30 mL) and tetrahydrofuran (30 mL) were added, and the mixture was refluxed and stirred for 5 hours. After completion of the reaction, it was neutralized with 1N aqueous hydrochloric acid and extracted with ethyl acetate (3 × 150 mL). The extract was washed with water (100 mL), dried over sodium sulfate, filtered, and the filtrate was concentrated to give compound (17-1, 3.50 g, 48% yield).

**Step G:** To compound **17-1** (3.5 g, 15.3 mmol) was added sodium acetate (5 equivalents), anhydrous acetic acid (40 mLl), and glacial acetic acid (20 mL) and stirred at 150 °C for 6 hours. After completion of the reaction, it was cooled to room temperature, diluted with water (150 mL), and extracted with ethyl acetate (3 × 120 mL). The extract was washed sequentially with aqueous sodium bicarbonate solution (100 mL) and brine (100 mL), the organic layer was dried over anhydrous sulfuric acid and filtered, and the filtrate was concentrated and separated by column chromatography to give the target compound (**17-2**, 1.77 g, 55% yield).

**Step H:** Compound **17-2** (1.77 g, 8.44 mol) was dissolved in anhydrous 1,4-dioxane (18 mL), then 4 M hydrochloric acid 1,4-dioxane solution (5 equivalents) was added and stirred under reflux for 16 hours. After completion of the reaction, the reaction solution was slowly added to 200 mL of water and extracted with ethyl acetate (100 mL × 3). The extract was washed with aqueous sodium bicarbonate solution (150 mL), the organic layer was dried over anhydrous sodium sulfate and filtered, and the filtrate was concentrated and separated by column chromatography to give the target compound (**17-3,** 1.16 g, 82% yield).

**Step I:** Compound **17-3** (1.16 g, 6.9 mmol) was dissolved in methanol (18 mL), cooled to 0 °C, and sodium borohydride (3 equivalents) was slowly added dropwise over 15 minutes. The reaction solution was stirred at room temperature for 2 hours, then cold water (20 mL) was added, and then extracted with dichloromethane (100 mL × 2). The extract was washed sequentially with water (50 mL) and brine (50 mL), the organic layer was dried over anhydrous sodium sulfate and filtered, and the filtrate was concentrated and separated by column chromatography to give the target compound **17-4** (0.918 g).

**Step J:** After dissolving compound **17-4** (0.918 g) in 1,4-dioxane (10 mL), 1,4-dioxane hydrochloric acid solution (5 equivalents) was added and stirred under reflux for 6 hours. After completion of the reaction, it was cooled to room temperature, diluted with water (30 mL), and extracted with ethyl acetate (3 × 120 mL). The extract was washed with an aqueous sodium bicarbonate solution (20 mL), the organic layer was dried over anhydrous sodium sulfate and filtered, and the filtrate was concentrated and separated by column chromatography to give the target compound (**17-5,** 0.641 g, 61% total yield from two steps).

**Step K:** Compound **17-5** (0.641 g, 4.2 mmol) was dissolved in anhydrous CS2 (10 mL), cooled to 0 °C, 4-methoxybenzoyl chloride (1.5 equiv) was added, and tin(IV) chloride (1.5 equiv) was added dropwise for 5 min. The reaction solution was stirred at room temperature for 24 hours, then diluted with cold water (200 mL), the resulting solid was filtered off, and the aqueous layer was extracted with dichloromethane (90 mL × 2). The extract was sequentially dried over 1M aqueous hydrochloric acid solution (20 mL), 1M aqueous sodium hydroxide solution (20 mL), and brine (30 mL), the organic layer was dried over anhydrous sodium sulfate, filtered, and the filtrate was concentrated and separated by column chromatography to give the target compound (**17-6,** 0.986 g, 78% yield).

**Step L:** Compound **17-6** (0.986 g, 3.2 mmol) was dissolved in anhydrous dichloromethane (10 mL), cooled to -78°C, and boron tribromide (1M dichloromethane solution, 5 equivalents) was added. The reaction solution was stirred at room temperature for 20 hours, then cold water (10 mL) was slowly added, and extracted with dichloromethane (30 mL × 2). The extract was washed sequentially with water (30 mL) and brine (30 mL), the organic layer was dried over anhydrous sodium sulfate and filtered, and the filtrate was concentrated and separated by column chromatography to give the target compound (17-7, 0.856 g, 91% yield).

**Step M:** Using compound **17-7,** the target compound **17** was obtained in a similar process to step E of Example 16.

¹H NMR (DMSO-d₆, 400 MHz) 7.94 (d, *J=* 1.7 Hz, 1H), 7.89 (s, 2H), 7.43 (dd, *J* = 8.5, 1.7 Hz, 1H), 7.34 (d, *J=* 8.4 Hz, 1H), 2.44 (s, 3H).

### Example 18. (5-bromo-2-methylbenzofuran-3-yl)(3,5-dibromo-4-hydroxyphenyl)methanone

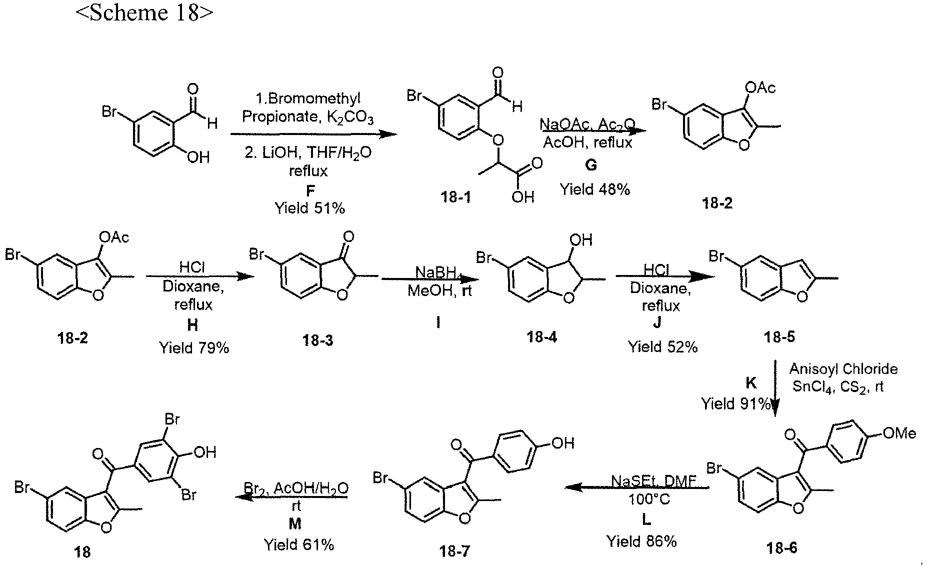

**Step F - Step M:** Compound **18** was obtained in a similar process to steps F-M of Example 17.

¹H NMR (DMSO-d₆, 400 MHz) 7.90 (s, 2H), 7.59 (dd, *J* = 6.8, 5.4 Hz, 2H), 7.47 (dd, *J* = 8.7, 2.0 Hz, 1H), 2.43 (s, 3H).

### Example 19. (6-chloro-2-methylbenzofuran-3-yl)(4-hydroxy-3,5-diiodophenyl)methanone

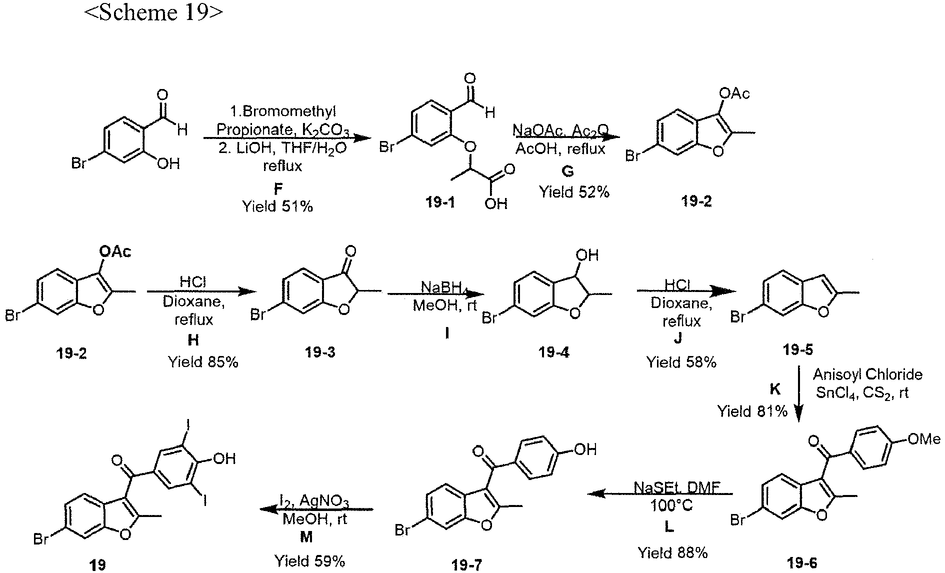

**Step F - Step L:** Compound **19-7** was obtained in a similar process to steps F-L of Example 17.

**Step M:** Compound **19** was obtained using compound **19-7** in a similar process to step E of Example 8.

¹H NMR (DMSO-d₆, 400 MHz) δ 8.07 (s, 2H), 7.94 (d, *J* = 1.7 Hz, 1H), 7.43 (dd, *J* = 8.3, 1.7 Hz, 1H), 7.33 (d, *J* = 8.4 Hz, 1H), 2.43 (s, 3H).

### Example 20. (3,5-dibromo-4-hydroxyphenyl)(2,5-diethylbenzofuran-3-yl)methanone

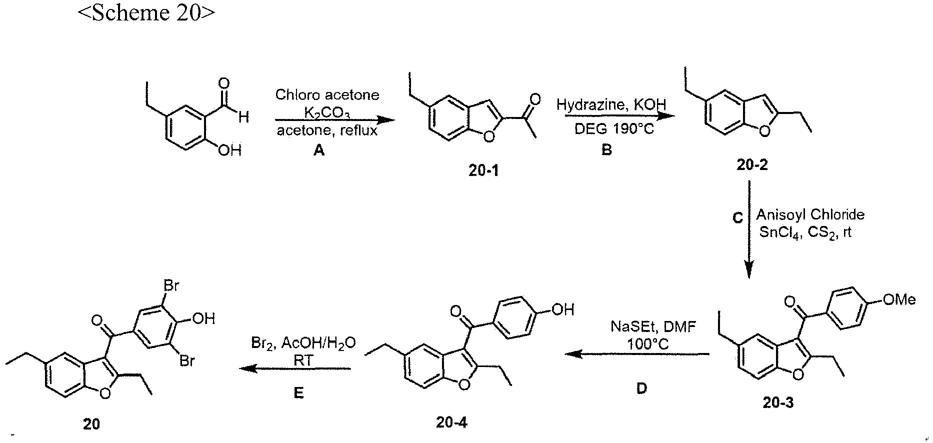

**Step A - Step E:** Compound **20** was obtained in a similar process to steps A-E of Example 1.

¹H NMR (DMSO-d₆, 400 MHz) δ 11.41 (s, 1H), 8.00 - 7.91 (m, 2H), 7.70 (dt, *J* = 8.4, 2.8 Hz, 1H), 7.36 (s, 1H), 7.09 (d, *J=* 8.5 Hz, 1H), 2.76 (dq, J = 20.2, 7.5 Hz, 4H), 1.24 (t, *J* = 7.5 Hz, 3H), 1.14 (t, *J=* 7.5 Hz, 3H).

### Example 21. (3,5-dibromo-4-hydroxyphenyl)(2,7-diethylbenzofuran-3-yl)methanone

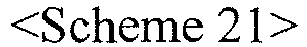

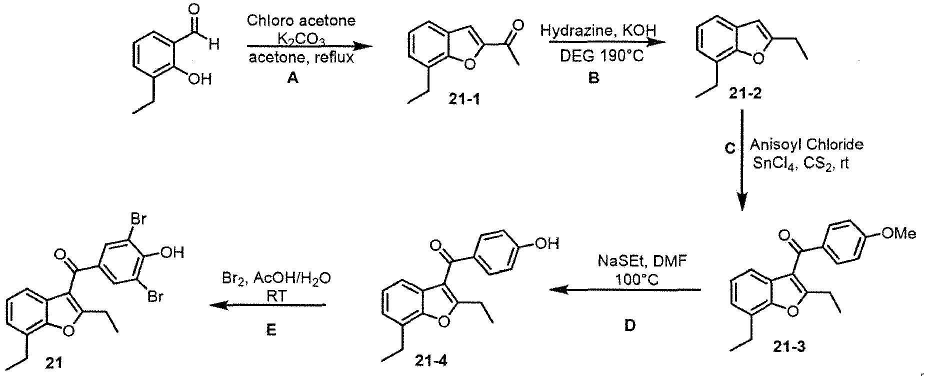

**Step A - Step E:** Compound **21** was obtained in a similar process to steps A-E of Example 1.

¹H NMR (DMSO-d₆, 400 MHz) δ 11.39 (s, 1H), 7.93 (d, *J* = 2.1 Hz, 1H), 7.67 (dd, *J* = 8.4, 2.1 Hz, 1H), 7.47 (d, *J* = 8.3 Hz, 1H), 7.16 (d, *J* = 8.4 Hz, 1H), 7.07 (d, *J* = 8.5 Hz, 1H), 3.01 (q, *J* = 7.6 Hz, 2H), 2.82 (q, *J* = 7.5 Hz, 2H), 1.26 (td, *J* = 7.5, 5.9 Hz, 6H).

### Example 22. (3,5-dibromo-4-hydroxyphenyl)(2-ethyl-6-(trifluoromethyl)benzofuran-3-yl)methanone

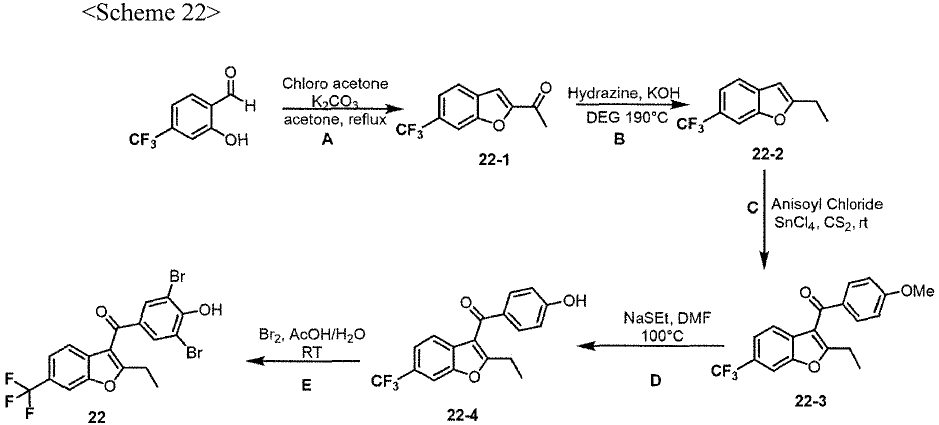

**Step A - Step E:** Compound **22** was obtained in a similar process to steps A-E of Example 1.

¹H NMR (DMSO-d₆, 400 MHz) δ 8.16 (s, 1H), 7.98 (s, 2H), 7.69 - 7.62 (m, 2H), 2.85 (q, *J* = 7.5 Hz, 2H), 1.29 (t, *J* = 7.5 Hz, 3H).

### Example 23. (3,5-dibromo-4-hydroxyphenyl)(2-ethyl-4-methylbenzofuran-3-yl)methanone

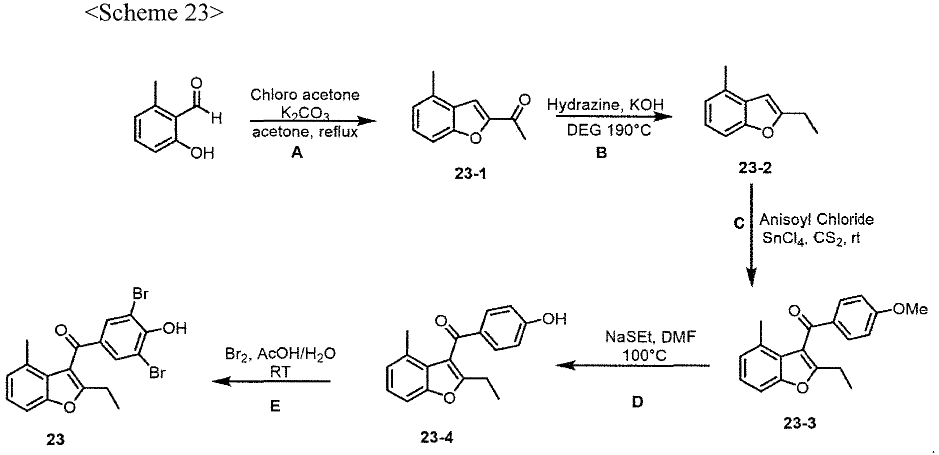

**Step A - Step E:** Compound **23** was obtained in a similar process to steps A-E of Example 1.

¹H NMR (DMSO-d₆, 400 MHz) δ 11.59 (s, 1H), 8.00 (d, *J=* 2.1 Hz, 1H), 7.69 (dd, *J* = 8.5, 2.2 Hz, 1H), 7.56 (d, *J=* 8.7 Hz, 1H), 7.48 (d, *J=* 8.7 Hz, 1H), 7.07 (d, *J=* 8.5 Hz, 1H), 2.62 (q, *J=* 7.5 Hz, 2H), 2.16 (s, 3H), 1.17 (t, *J=* 7.5 Hz, 3H).

### Example 24. (3,5-dibromo-4-hydroxyphenyl)(2-ethyl-4-fluorobenzofuran-3-yl)methanone

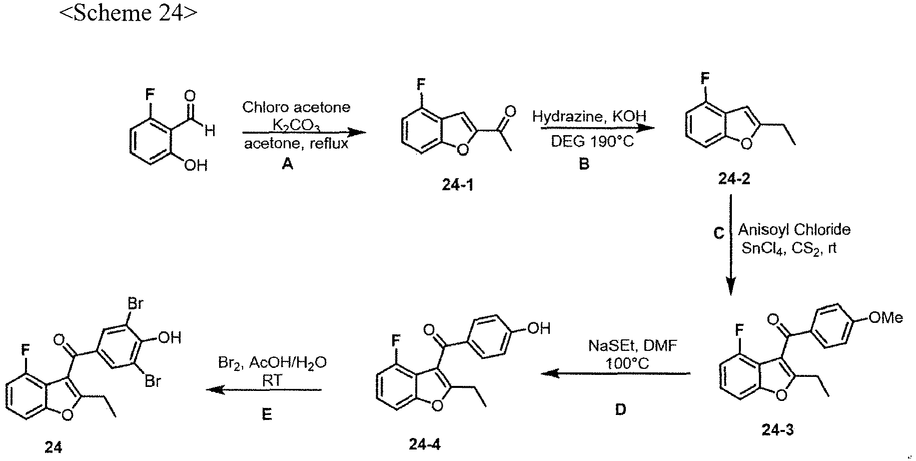

**Step A - Step E:** Compound **24** was obtained in a similar process to steps A-E of Example 1.

¹H NMR (DMSO-d₆, 400 MHz) δ 11.59 (s, 1H), 8.00 (d, *J* = 2.1 Hz, 1H), 7.69 (dd, *J* = 8.5, 2.2 Hz, 1H), 7.56 (d, *J=* 8.7 Hz, 1H), 7.48 (d, *J=* 8.7 Hz, 1H), 7.07 (d, *J=* 8.5 Hz, 1H), 2.62 (q, *J=* 7.5 Hz, 2H), 2.16 (s, 3H), 1.17 (t, *J=* 7.5 Hz, 3H).

### <Experimental example>

### 1. Evaluation of the compounds

### (1) Evaluation of HSP47 activity

Fibrils were formed by adding 180 ul of phosphate-buffered saline (PBS, pH 7.4) solution to 20 ul of collagen dissolved in acidic solution (collagen solution, UK), which was measured at a wavelength of 340 nm. HSP47 (GenScript US) was added at a concentration of 9.45 µg/ml to assess HSP47 activity as the degree to which it inhibits fibril formation. Test compound was added with concentrations of 100 µM to 1 µM and the inhibitory activity of HSP47 was calculated as a percentage or EC₅₀.

### (2) Sirius red assay

Liver stellate LX-2 (Elabscience CH), lung epithelial A549 (ATCC US), or skin KEL FIB (ATCC, US) cells were cultured in 24-well tissue culture plates for 18 hours, followed by TGF-beta 10 ng/ml treatment, and then incubated with test compound for 24 hours. Cells were washed with PBS, fixed with Bouin's solution, and washed twice with distilled water. The fixed cells were stained with Sirius red for 2 hours to observe cellular morphological changes, and then washed with 0.01 N HCl solution. Collagen-bound Sirius red was extracted with 0.1 N NaOH solution, which was then quantified at a wavelength of 570 nm. The inhibitory efficacy of each compound on collagen production was shown as a percentage or EC₅₀.

### (3) Inhibition of MCP-1 (Monocyte Chemoattractant Protein-1) Secretion

LX-2 cells, a hepatic stellate cell line, were cultured in 24-well tissue culture plates for 18 hours, followed by TGF-beta 10 ng/ml treatment, and then incubated with 30 to 1 µM of the test compound for 24 hours. The secreted MCP-1 protein in the cell culture medium was quantified using ELISA reagents (RnD, USA). The EC₅₀ value was calculated from the quantitative value of MCP-1 resulting from the treatment of each test compound using prism software (Graphpad, ver 9.4 USA).

### (4) Statistical analysis

The results of this experiment are analyzed using parametric multiple comparison procedures or non-parametric multiple comparison procedures, assuming normality of the data.

If the results of the parametric One-way ANOVA were significant, post-hoc tests are performed using Dunnett's multiple comparison test, and if the results of the non-parametric Kruskal-Wallis' H-test analysis were significant, post-hoc tests are performed using Dunn's multiple comparison test.

Statistical analysis is performed using Prism (GraphPad Software Inc., USA), and a p-value of less than 0.05 is considered statistically significant.

### 2. Results

### (1) Inhibition of HSP47 function

The results of inhibition of HSP47 protein function are listed in Table 1 below.

**[Table 1]**

| **Example** | HSP47 inhibition **EC₅₀, µM** | **% inhibition at 100µM** |
|---|---|---|
| 1 | - | 23.31 |
| 2 | 40.75 | - |
| 3 | - | 26.28 |
| 4 | 73.04 | - |
| 5 | 37.76 | - |
| 6 | 80.82 | - |
| 7 | - | 18.15 |
| 8 | 37.1 | - |
| 9 | - | 52.38 |
| 10 | - | 41.77 |
| 11 | - | 59.24 |
| 12 | 73.14 | - |
| 13 | 49.14 | - |
| 14 | 15.89 | - |
| 15 | 48.26 | - |
| 16 | 31.4 | - |
| 17 | - | 19.5 |
| 18 | - | 75.2 |
| 19 | - | 58.19 |
| 20 | 49.23 | - |
| 21 | 5.524 | - |
| 22 | 32.09 | - |
| 23 | 71 | - |
| 24 | 104.5 | - |

### (2) Inhibition of hepatic stellate cell T6 fibrosis

The results of inhibition of hepatic stellate cell fibrosis are listed in Table 2 below.

**[Table 2]**

| **Example** | **LX-2 cell EC₅₀, µM** | **% inhibition at 10µM** |
|---|---|---|
| 1 | 2.998 | - |
| 2 | - | 65.10 |
| 3 | 1.374 | - |
| 4 | 5.552 | - |
| 5 | 4.727 | - |
| 6 | 17.14 | - |
| 7 | 10.88 | - |
| 8 | - | 57.9 |
| 9 | - | 111.12 |
| 10 | - | 127.72 |
| 11 | - | 78.30 |
| 12 | 8.151 | - |
| 14 | - | 90.62 |
| 17 | 3.699 | - |
| 18 | 4.667 | - |
| 19 | - | 72.6 |
| 20 | 4.018 | - |
| 21 | 3.067 | - |
| 22 | 2.805 | - |
| 23 | 3.393 | - |
| 24 | 3.595 | - |

### (3) Inhibition of lung epithelial cell A549 fibrosis

The results of inhibition of lung epithelial cell fibrosis are listed in Table 3 below.

**[Table 3]**

| **Example** | **% inhibition at 30µM** | **% inhibition at 10µM** |
|---|---|---|
| 13 | 72.46 | 65.16% |
| 15 | 96.25 | - |
| 16 | - | 54.315 |

### (4) Inhibition of dermal fibroblast KEL FIB fibrosis

The results of inhibition of skin fibroblast fibrosis are listed in Table 4 below.

**[Table 4]**

| **Example** | **KEL FIB cell EC**₅₀ **µM** |
|---|---|
| 9 | 3.281 |

### (5) Inhibition of MCP-1 secretion in LX-2 astrocyte

The results of inhibition of MCP-1 secretion in LX-2 hepatic stellate cells by test compound treatment are listed in Table 5 below.

**[Table 5]**

| **Example** | **LX-2 cell EC**₅₀ **µM** |
|---|---|
| 1 | 4.61 |
| 3 | 2.211 |
| 4 | 8.687 |
| 5 | 3.557 |
| 6 | 10.16 |
| 7 | 2.85 |
| 17 | 3.733 |
| 18 | 3.008 |
| 20 | 2.102 |
| 21 | 2.801 |

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. A benzofuranyl hydroxyphenyl methanone derivative compound represented by the following Formula I, or a pharmaceutically acceptable salt thereof, wherein: 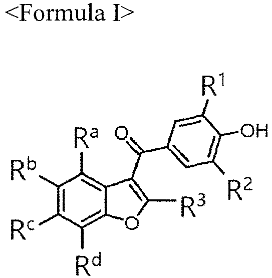
R¹ and R² are halogen,
R³ is hydrogen or C₁-C₃ alkyl,
R^{a}, R^{b} and R^{d} are independently hydrogen, C₁-C₃ alkyl or halogen,
R^{c} is hydrogen, C₁-C₃ alkyl, halogen or C₁-C₃ haloalkyl.

2. The compound or a pharmaceutically acceptable salt thereof of Claim 1, **characterized in that** R¹ and R² are bromo or iodo.

3. The compound or a pharmaceutically acceptable salt thereof of Claim 1, **characterized in that** R³ is a substituent selected from the group consisting of hydrogen, methyl and ethyl.

4. The compound or a pharmaceutically acceptable salt thereof of Claim 1, **characterized in that** R^{a} is a substituent selected from the group consisting of hydrogen, methyl, fluoro and chloro.

5. The compound or a pharmaceutically acceptable salt thereof of Claim 1, **characterized in that** R^{b} is a substituent selected from the group consisting of hydrogen, methyl, ethyl and bromo.

6. The compound or a pharmaceutically acceptable salt thereof of Claim 1, **characterized in that** R^{c} is a substituent selected from the group consisting of hydrogen, methyl, ethyl, trifluoromethyl, chloro and bromo.

7. The compound or a pharmaceutically acceptable salt thereof of Claim 1, **characterized in that** R^{d} is a substituent selected from the group consisting of hydrogen, methyl, ethyl, fluoro, chloro and bromo.

8. The compound or a pharmaceutically acceptable salt thereof of Claim 1, **characterized in that** the benzofuranyl hydroxyphenyl methanone derivative compound represented by Formula I is selected from the group consisting of:
(4-chloro-2-ethylbenzofuran-3-yl)(3,5-dibromo-4-hydroxyphenyl)methanone,
(3,5-dibromo-4-hydroxyphenyl)(2-ethyl-5-methylbenzofuran-3-yl)methanone,
(3,5-dibromo-4-hydroxyphenyl)(2,6-diethylbenzofuran-3-yl)methanone,
(3,5-dibromo-4-hydroxyphenyl)(2-ethyl-7-methylbenzofuran-3-yl)methanone,
(7-chloro-2-ethylbenzofuran-3-yl)(3,5-dibromo-4-hydroxyphenyl)methanone,
(7-bromo-2-ethylbenzofuran-3-yl)(3,5-dibromo-4-hydroxyphenyl)methanone,
(3,5-dibromo-4-hydroxyphenyl)(2-ethyl-7-fluorobenzofuran-3-yl)methanone,
(2-ethyl-5-methylbenzofuran-3-yl)(4-hydroxy-3,5-diiodophenyl)methanone,
(5-bromo-2-ethylbenzofuran-3-yl)(4-hydroxy-3,5-diiodophenyl)methanone,
(2-ethyl-6-methylbenzofuran-3-yl)(4-hydroxy-3,5-diiodophenyl)methanone,
(6-bromo-2-ethylbenzofuran-3-yl)(4-hydroxy-3,5-diiodophenyl)methanone,
(6-chloro-2-ethylbenzofuran-3-yl)(3,5-dibromo-4-hydroxyphenyl)methanone,
(2-ethyl-7-methylbenzofuran-3-yl)(4-hydroxy-3,5-diiodophenyl)methanone,
(7-chloro-2-ethylbenzofuran-3-yl)(4-hydroxy-3,5-diiodophenyl)methanone,
(7-bromo-2-ethylbenzofuran-3-yl)(4-hydroxy-3,5-diiodophenyl)methanone,
benzofuran-3-yl(3,5-dibromo-4-hydroxyphenyl)methanone,
(6-bromo-2-methylbenzofuran-3-yl)(3,5-dibromo-4-hydroxyphenyl)methanone,
(5-bromo-2-methylbenzofuran-3-yl)(3,5-dibromo-4-hydroxyphenyl)methanone,
(6-chloro-2-methylbenzofuran-3-yl)(4-hydroxy-3,5-diiodophenyl)methanone,
(3,5-dibromo-4-hydroxyphenyl)(2,5-diethylbenzofuran-3-yl)methanone,
(3,5-dibromo-4-hydroxyphenyl)(2,7-diethylbenzofuran-3-yl)methanone,
(3,5-dibromo-4-hydroxyphenyl)(2-ethyl-6-(trifluoromethyl)benzofuran-3-yl)methanone,
(3,5-dibromo-4-hydroxyphenyl)(2-ethyl-4-methylbenzofuran-3-yl)methanone, and
(3,5-dibromo-4-hydroxyphenyl)(2-ethyl-4-fluorobenzofuran-3-yl)methanone.

9. A pharmaceutical composition for use in a method of inhibiting HSP47 comprising the benzofuranyl hydroxyphenyl methanone derivative compound, or a pharmaceutically acceptable salt thereof of any one of Claims 1 to 8 as an active ingredient.

10. The pharmaceutical composition for use in a method of inhibiting HSP47 of Claim 9, **characterized in that** inhibiting HSP47 is preventing or treating fibrosis.

11. The pharmaceutical composition for use in a method of inhibiting HSP47 of Claim 10, **characterized in that** the fibrosis is one or more selected from the group consisting of liver cirrhosis, pulmonary fibrosis, keloid, hypertrophic scar and renal fibrosis (glomerulosclerosis).

12. The pharmaceutical composition for use in a method of inhibiting HSP47 of Claim 9, **characterized in that** inhibiting HSP47 is preventing or treating cancer.

13. The pharmaceutical composition for use in a method of inhibiting HSP47 of Claim 12, **characterized in that** the cancer is one or more selected from the group consisting of breast cancer, colon cancer and cancer-related fibrosis.

14. The pharmaceutical composition for use in a method of inhibiting HSP47 of Claim 9, **characterized in that** inhibiting HSP47 is preventing or treating inflammatory disease.

15. The pharmaceutical composition for use in a method of inhibiting HSP47 of Claim 14, **characterized in that** the inflammatory disease is one or more selected from the group consisting of liver cancer, prostate cancer, melanoma, ovarian cancer, neuroinflammatory diseases, arteriosclerosis, organ transplantation, dermatitis, atopic dermatitis, asthma, conjunctivitis, periodontitis, rhinitis, otitis media, pharyngitis, tonsillitis, pneumonia, stomach ulcer, pancreatitis, gastritis, psoriasis, nephropathy, neuroinflammatory diseases, myasthenia gravis, Crohn's disease, inflammatory bowel disease, colitis, gout, ankylosing spondylitis, lupus, fibromyalgia, psoriasis, rheumatoid arthritis, osteoarthritis, osteoporosis, hepatitis, cystitis, nephritis, Sjögren's syndrome and multiple sclerosis.

16. Method for preparing a benzofuranyl hydroxyphenyl methanone derivative compound represented by Formula I below, or a pharmaceutically acceptable salt thereof, comprising:
preparing a compound of Formula I-2 below by a nucleophilic substitution reaction from a compound of Formula 1-1 below;
reducing the compound of Formula I-2 obtained above to prepare a compound of Formula I-3 below;
reacting the compound of Formula 1-3 obtained above with 4-anisoyl chloride to prepare a compound of Formula I-4 below;
removing the methyl of the methoxy of the compound of Formula I-4 obtained above to prepare a compound of Formula I-5 below having a hydroxy substituent; and
obtaining a compound of Formula I below from the compound of Formula I-5 obtained above: 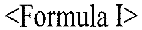 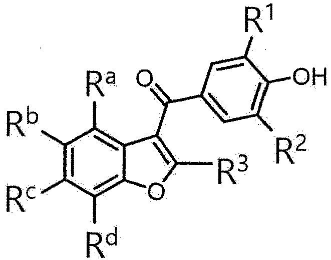 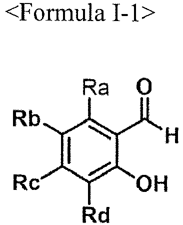 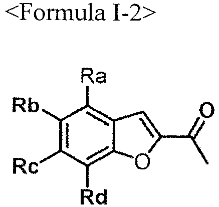 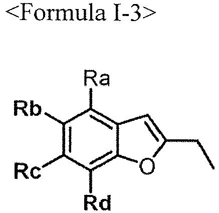 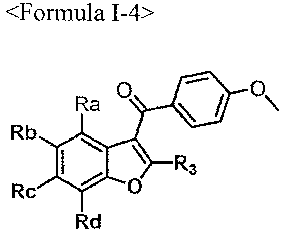 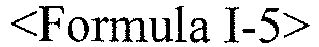 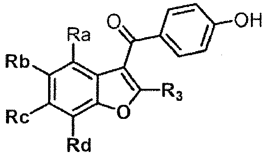
wherein in Formula I, Formula I-1, Formula I-2, Formula I-3, Formula I-4 and Formula I-5, R¹, R², R³, R^{a}, R^{b}, R^{c} and R^{d} are the same as defined in Claim 1.
